(19) Europäisches Patentamt
European Patent Office
Office européen des brevets

(11) Veröffentlichungsnummer: **0 528 212 A1**

# (12) EUROPÄISCHE PATENTANMELDUNG

(21) Anmeldenummer: **92112795.7**

(22) Anmeldetag: **27.07.92**

(51) Int. Cl.5: **C07D 239/47**, A01N 47/36, C07D 239/52, C07D 239/56, C07D 251/16, C07D 251/46, C07D 239/42, C07D 251/22

(30) Priorität: **09.08.91 DE 4126423**
**11.02.92 DE 4203875**

(43) Veröffentlichungstag der Anmeldung:
**24.02.93 Patentblatt 93/08**

(84) Benannte Vertragsstaaten:
**BE CH DE DK ES FR GB IT LI NL**

(71) Anmelder: **BAYER AG**

**W-5090 Leverkusen 1 Bayerwerk(DE)**

(72) Erfinder: **Riebel, Hans-Jochem, Dr.**
**In der Beek 92**
**W-5600 Wuppertal 1(DE)**
Erfinder: **Santel, Hans-Joachim, Dr.**
**Grünstrasse 9a**
**W-5090 Leverkusen 1(DE)**
Erfinder: **Lürssen, Klaus, Dr.**
**August-Kierspel-Strasse 145**
**W-5060 Bergisch-Gladbach 2(DE)**
Erfinder: **Schmidt, Robert R., Dr.**
**Im Waldwinkel 110**
**W-5060 Bergisch-Gladbach 2(DE)**

## (54) N-Azinyl-N'-(2-ethylsulfinyl-phenylsulfonyl)Harnstoffe als Herbizide.

(57) Die Erfindung betrifft neue N-Azinyl-N'-(2-ethylsulfinyl-phenylsulfonyl)-harnstoffe der Formel (I),

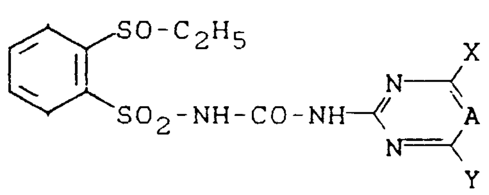

(I)

in welcher

A für Stickstoff oder eine CH-Gruppierung steht,

X für Wasserstoff, Halogen oder für jeweils gegebenenfalls durch Halogen substituiertes Alkyl, Alkoxy oder Alkylthio mit jeweils 1 bis 3 Kohlenstoffatomen steht und

Y für jeweils gegebenenfalls durch Halogen substituiertes Alkyl oder Alkoxy mit jeweils 1 bis 3 Kohlenstoffatomen steht,

sowie Salze von Verbindungen der Formel (I), Verfahren zur Herstellung dieser Verbindungen und ihrer Salze sowie deren Verwendung als Herbizide.

EP 0 528 212 A1

Die Erfindung betrifft neue N-Azinyl-N'-(2-ethylsulfinyl-phenylsulfonyl)-harnstoffe, Verfahren zu ihrer Herstellung und ihre Verwendung als Herbizide.

Es ist bekannt, daß bestimmte Arylsulfonylharnstoffe mit über Schwefel gebundenen Substituenten, wie z.B. N-(4,6-Dimethoxy-s-triazin-2-yl)-N'-(2-propylthio-phenylsulfonyl)-harnstoff (vgl. US-P 4818277) oder N-(4,6-Dimethylpyrimidin-2-yl)-N'-(2-methylsulfinyl-phenylsulfonyl)-harnstoff (vgl. US-P 4169719; vgl. auch EP-A 101308, EP-A 35893) herbizide Eigenschaften aufweisen. Die Herbizidwirkung dieser bekannten Verbindungen ist jedoch nicht in allen Belangen zufriedenstellend.

Es wurden nun neue N-Azinyl-N'-(2-ethylsulfinyl-phenylsulfonyl)-harnstoffe der allgemeinen Formel (I)

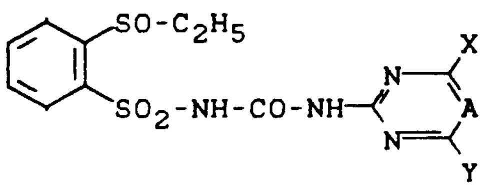

(I)

in welcher

A für Stickstoff oder eine CH-Gruppierung steht,

X für Wasserstoff, Halogen oder für jeweils gegebenenfalls durch Halogen substituiertes Alkyl, Alkoxy oder Alkylthio mit jeweils 1 bis 3 Kohlenstoffatomen steht und

Y für jeweils gegebenenfalls durch Halogen substituiertes Alkyl oder Alkoxy mit jeweils 1 bis 3 Kohlenstoffatomen steht,

sowie Salze van Verbindungen der Formel (I) gefunden.

Man erhält die neuen N-Azinyl-N'-(2-ethylsulfinyl-phenylsulfonyl)-harnstoffe der allgemeinen Formel (I), wenn man

N-Azinyl-N'-(2-ethylthio-phenylsulfonyl)-harnstoffe der allgemeinen Formel (II)

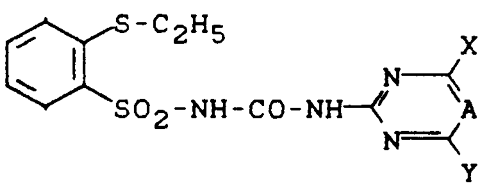

(II)

in welcher

A, X und Y die oben angegebenen Bedeutungen haben,

mit Hydrogenperoxid ($H_2O_2$) in Gegenwart eines Verdünnungsmittels umsetzt

und gegebenenfalls die so erhaltenen Produkte nach üblichen Methoden in Salze überführt.

Die neuen N-Azinyl-N'-(2-ethylsulfinyl-phenylsulfonyl)-harnstoffe der Formel (I) zeichnen sich durch starke herbizide Wirksamkeit aus.

Überraschenderweise zeigen die neuen Verbindungen der Formel (I) erheblich stärkere Wirkung als die nach Struktur und Wirkprofil vergleichbaren bekannten Verbindungen N-(4,6-Dimethoxy-s-triazin-2-yl)-N'-(2-propylthio-phenylsulfonyl)-harnstoff und N-(4,6-Dimethylpyrimidin-2-yl)-N'-(2-methylsulfinyl-phenylsulfonyl)-harnstoff.

Gegenstand der Erfindung sind vorzugsweise Verbindungen der Formel (I), in welcher

A für Stickstoff oder eine CH-Gruppierung steht,

X für Wasserstoff, Fluor, Chlor, Brom oder für jeweils gegebenenfalls durch Fluor und/oder Chlor substituiertes Alkyl, Alkoxy oder Alkylthio mit jeweils 1 oder 2 Kohlenstoffatomen steht und

Y für jeweils gegebenenfalls durch Fluor und/oder Chlor substituiertes Alkyl oder Alkoxy mit jeweils 1 oder 2 Kohlenstoffatomen steht.

Die Erfindung betrifft weiter vorzugsweise Salze, die man aus Verbindungen der Formel (I) und Basen, wie z.B. Natrium-, Kalium- oder Calcium-hydroxid, -hydrid, -amid oder -carbonat, Natrium- oder Kalium-$C_1$-$C_4$-alkanolaten, Ammoniak, $C_1$-$C_4$-Alkylaminen, Di-($C_1$-$C_4$-alkyl)-aminen oder Tri-($C_1$-$C_4$-alkyl)-aminen, erhält.

Die Erfindung betrifft insbesondere Verbindungen der Formel (I), in welcher

A für Stickstoff oder eine CH-Gruppierung steht,

X für Wasserstoff, Chlor, Methyl, Ethyl, Methoxy, Ethoxy, Difluormethoxy oder Methylthio steht und

Y für Methyl, Ethyl, Trifluormethyl, Methoxy, Ethoxy oder Difluormethoxy steht.

Verwendet man beispielsweise N-(4,6-Dimethoxy-pyrimidin-2-yl)-N'-(2-ethylthio-phenylsulfonyl)-harnstoff als Ausgangsstoff, so kann der Reaktionsablauf durch das folgende Formelschema skizziert werden:

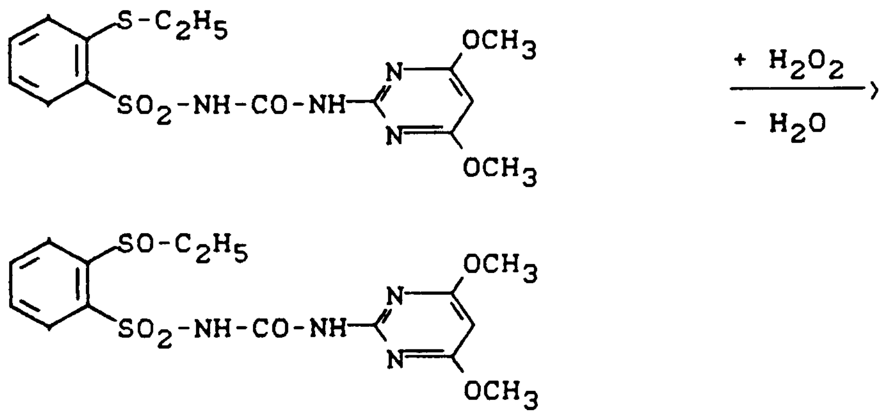


Die beim erfindungsgemäßen Verfahren zur Herstellung von Verbindungen der Formel (I) als Ausgangsstoffe zu verwendenden N-Azinyl-N'-(2-ethylthio-phenylsulfonyl)-harnstoffe sind durch die Formel (II) allgemein definiert.

In Formel (II) haben A, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, X und Y angegeben wurden.

Die Ausgangsstoffe der Formel (II) sind bekannt und/oder können nach an sich bekannten Verfahren hergestellt werden (vgl. US-P 4127405, US-P 4169719).

Das erfindungsgemäße Verfahren wird unter Verwendung von Hydrogenperoxid durchgeführt. Das Hydrogenperoxid wird vorzugsweise als wäßrige Lösung eingesetzt, welche vorzugsweise zwischen 5% und 55%, insbesondere zwischen 10% und 50% $H_2O_2$ enthält.

Das erfindungsgemäße Verfahren wird in Gegenwart eines Verdünnungsmittels durchgeführt. Es kommen dabei praktisch alle bei Oxidationsreaktionen üblichen Verdünnungsmittel in Betracht. Bevorzugte Verdünnungsmittel für das erfindungsgemäße Verfahren sind Carbonsäuren, wie Ameisensäure, Essigsäure und Propionsäure, insbesondere Essigsäure.

Die Reaktionstemperaturen können bei dem erfindungsgemäßen Verfahren in einem größeren Bereich variiert werden. Im allgemeinen arbeitet man bei Temperaturen zwischen 0 °C und 100 °C, vorzugsweise bei Temperaturen zwischen 10 °C und 60 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man auf 1 Mol Ausgangsverbindung der Formel (II) im allgemeinen zwischen 1,0 und 2,0 Mol, vorzugsweise zwischen 1,1 und 1,5 Mol, Hydrogenperoxid ein.

Die Reaktionskomponenten werden vorzugsweise wie folgt zur Umsetzung gebracht:
Zunächst wird der Sulfonylharnstoff der Formel (II) mit dem Verdünnungsmittel vermischt und unter Rühren dieser Mischung wird das Hydrogenperoxid langsam eindosiert. Das Reaktionsgemisch wird dann noch, gegebenenfalls bei erhöhter Temperatur bis zum Ende der Umsetzung gerührt. Nach Erkalten der Mischung und gegebenenfalls Verdünnen mit Wasser wird das kristalline Produkt durch Absaugen isoliert.

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Verdünnungsmittel, wie z.B. Methylenchlorid, tert-Butyl-methylether oder Toluol, und Zugabe einer geeigneten Base. Die Salze können dann -gegebenenfalls nach längerem Rühren - durch Einengen oder Absaugen isoliert werden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle

Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen, sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von

Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkänsauren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Herstellungsbeispiele:

Beispiel 1:

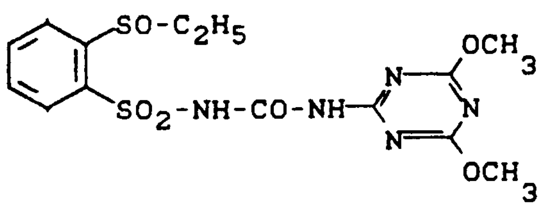

Eine Mischung aus 16 g (0,04 Mol) N-(4,6-Dimethoxy-s-triazin-2-yl)-N'-(2-ethylthio-phenylsulfonyl)-harnstoff, 70 ml Essigsäure und 6 ml einer 30%igen wäßrigen Lösung von Hydrogenperoxid (0,06 Mol $H_2O_2$) wird 18 Stunden bei 22° C gerührt. Nach Zugabe von 120 ml Wasser wird das kristallin angefallene Produkt durch Absaugen isoliert.

Man erhält 14 g (84% der Theorie) N-(4,6-Dimethoxy-s-triazin-2-yl)-N'-(2-ethylsulfinyl-phenylsulfonyl)-harnstoff vom Schmelzpunkt 165 ° C.

Salze von Verbindungen der Formel (I):

Beispiel 4a

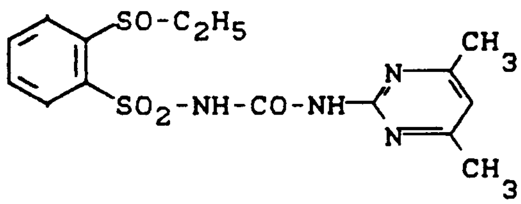

-Na-Salz

0,5 g (0,01 Mol) 80%iges Natriumhydroxid-Pulver werden unter Rühren zu einer Mischung aus 3,9 g (0,01 Mol) N-(4,6-Dimethyl-pyrimidin-2-yl)-N'-(2-ethylsulfinyl-phenylsulfonyl)-harnstoff und 100 ml Toluol gegeben. Das Gemisch wird 15 Stunden bei 20 ° C gerührt; anschließend wird das kristalline Produkt durch Absaugen isoliert.

Man erhält 4,0 g (99% der Theorie) N-(4,6-Dimethyl-pyrimidin-2-yl)-N'-(2-ethylsulfinyl-phenylsulfonyl)-harnstoff-Na-Salz vom Schmelzpunkt 105 ° C (unter Zersetzung).

Analog Beispiel 1 bzw. Beispiel 4a und entsprechend der allgemeinen Beschreibung des erfindungsgemäßen Herstellungsverfahrens können beispielsweise auch die in der nachstehenden Tabelle 1 aufgeführten Verbindungen der Formel (I) bzw. ihre Salze hergestellt werden.

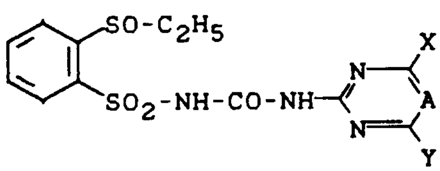

(I)

Tabelle 1: Beispiele für die Verbindungen der Formel (I) und ihre Salze

| Bsp.-Nr. | A | X | Y | Salz | Schmelzpunkt (°C) |
|---|---|---|---|---|---|
| 2 | N | $OCH_3$ | $CH_3$ | - | 109 |
| 3 | CH | $OCH_3$ | $OCH_3$ | - | 187 |
| 1a | N | $OCH_3$ | $OCH_3$ | Na-Salz | 190 |
| 2a | N | $OCH_3$ | $CH_3$ | Na-Salz | 200 |
| 3a | CH | $OCH_3$ | $OCH_3$ | Na-Salz | 185 |
| 4 | CH | $CH_3$ | $CH_3$ | - | 135 |
| 5 | CH | $OCH_3$ | $CH_3$ | - | 182 |
| 5a | CH | $OCH_3$ | $CH_3$ | Na-Salz | 148 |
| 6 | CH | Cl | $OCH_3$ | - | 204 |
| 6a | CH | Cl | $OCH_3$ | Na-Salz | 155 |
| 7 | CH | Cl | $OC_2H_5$ | - | 148 |
| 7a | CH | Cl | $OC_2H_5$ | Na-Salz | 80 |
| 8 | N | $OCH_3$ | $OC_2H_5$ | - | 167 |
| 8a | N | $OCH_3$ | $OC_2H_5$ | Na-Salz | 137 |
| 9 | CH | H | $CH_3$ | - | 192 |
| 9a | CH | H | $CH_3$ | Na-Salz | 170 |
| 10 | CH | $OC_2H_5$ | $OC_2H_5$ | - | 142 |
| 10a | CH | $OC_2H_5$ | $OC_2H_5$ | Na-Salz | 140 |

Tabelle 1 - Fortsetzung

| Bsp.-Nr. | A | X | Y | Salz | Schmelzpunkt (°C) |
|---|---|---|---|---|---|
| 11 | N | $CH_3$ | $CH_3$ | - | 146 |
| 11a | N | $CH_3$ | $CH_3$ | Na-Salz | 170 |
| 12 | CH | $C_2H_5$ | $OCH_3$ | - | |
| 13 | CH | $OC_2H_5$ | $CH_3$ | - | |
| 14 | CH | $OCH_3$ | $CF_3$ | - | |
| 15 | N | $OCH_3$ | $CF_3$ | - | |
| 16 | N | $OC_2H_5$ | $CH_3$ | - | |
| 17 | N | $C_2H_5$ | $OCH_3$ | - | |
| 18 | CH | $OCHF_2$ | $OCHF_2$ | - | |
| 19 | CH | $SCH_3$ | $CH_3$ | - | |
| 20 | CH | $SCH_3$ | $C_2H_5$ | - | |
| 21 | N | $SCH_3$ | $CH_3$ | - | |
| 22 | CH | Cl | $CH_3$ | - | |
| 23 | N | $SCH_3$ | $OCH_3$ | - | |
| 24 | N | Cl | $CH_3$ | - | |

Anwendungsbeispiele:

In den folgenden Anwendungsbeispielen werden die nachstehend aufgeführten Verbindungen als Vergleichssubstanzen herangezogen:

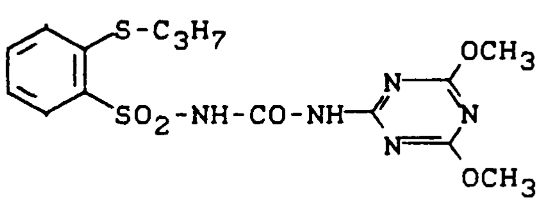

(A)

N-(4,6-Dimethoxy-s-triazin-2-yl)-N'-(2-propylthio-phenylsulfonyl)-harnstoff (bekannt aus US-P 4818277);

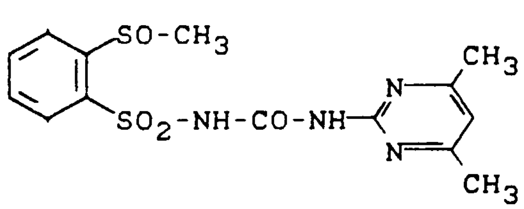

(B)

N-(4,6-Dimethyl-pyrimidin-2-yl)-N'-(2-methylsulfinylphenylsulfonyl)-harnstoff (bekannt aus US-Patent 4169719).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß folgender Herstellungsbeispiele: 2a, 3, 3a, 4, 4a, 5 und 5a.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß folgender Herstellungsbeispiele: 1, 2 und 3.

**Patentansprüche**

**1.** N-Azinyl-N'-(2-ethylsulfinyl-phenylsulfonyl)-harnstoffe der allgemeinen Formel (I),

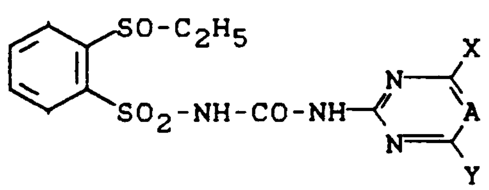

(I)

in welcher

A für Stickstoff oder eine CH-Gruppierung steht,

X für Wasserstoff, Halogen oder für jeweils gegebenenfalls durch Halogen substituiertes Alkyl, Alkoxy oder Alkylthio mit jeweils 1 bis 3 Kohlenstoffatomen steht und

Y für jeweils gegebenenfalls durch Halogen substituiertes Alkyl oder Alkoxy mit jeweils 1 bis 3 Kohlenstoffatomen steht,

sowie Salze von Verbindungen der Formel (I).

**2.** Harnstoffe der Formel (I) und deren Salze gemäß Anspruch 1, dadurch gekennzeichnet, daß darin

A für Stickstoff oder eine CH-Gruppierung steht,

X für Wasserstoff, Fluor, Chlor, Brom oder für jeweils gegebenenfalls durch Fluor und/oder Chlor substituiertes Alkyl, Alkoxy oder Alkylthio mit jeweils 1 oder 2 Kohlenstoffatomen steht und

Y für jeweils gegebenenfalls durch Fluor und/oder Chlor substituiertes Alkyl oder Alkoxy mit jeweils 1 oder 2 Kohlenstoffatomen steht.

**3.** Harnstoffe der Formel (I) und deren Salze gemäß Anspruch 1, dadurch gekennzeichnet, daß darin

A für Stickstoff oder eine CH-Gruppierung steht,

X für Wasserstoff, Chlor, Methyl, Ethyl, Methoxy, Ethoxy, Difluormethoxy oder Methylthio steht und

Y für Methyl, Ethyl, Trifluormethyl, Methoxy, Ethoxy oder Difluormethoxy steht.

**4.** Verfahren zur Herstellung von Harnstoffen der Formel (I) und ihrer Salze gemäß Anspruch 1, dadurch gekennzeichnet, daß man N-Azinyl-N'-(2-ethylthio-phenylsulfonyl)-harnstoffe der allgemeinen Formel (II)

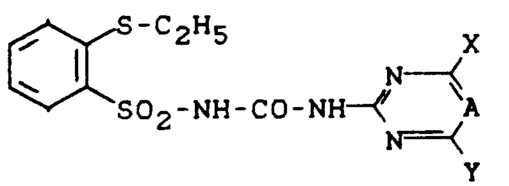

(II)

in welcher

A, X und Y die in Anspruch 1 angegebenen Bedeutungen haben,

mit Hydrogenperoxid ($H_2O_2$) in Gegenwart eines Verdünnungsmittels umsetzt und gegebenenfalls die so erhaltenen Produkte nach üblichen Methoden in Salze überführt.

**5.** Herbizide Mittel, gekennzeichnet durch einen Gehalt an mindestens einem Harnstoff der Formel (I) oder einem von dessen Salzen gemäß Anspruch 1.

**6.** Verfahren zur Bekämpfung von Unkräutern, dadurch gekennzeichnet, daß man Harnstoffe der Formel (I) oder deren Salze gemäß Anspruch 1 auf Unkräuter und/oder ihren Lebensraum einwirken läßt.

**7.** Verwendung von Harnstoffen der Formel (I) oder deren Salze gemäß Anspruch 1 zur Bekämpfung von Unkräutern.

**8.** Verfahren zur Herstellung von herbiziden Mitteln, dadurch gekennzeichnet, daß man Harnstoffe der Formel (I) oder deren Salze gemäß Anspruch 1 mit Streckmitteln und/ oder oberflächenaktiven Mitteln vermischt.

**9.** N-(4,6-Dimethoxy-pyrimidin-2-yl)-N'-(2-ethylsulfinyl-phenylsulfonyl)-harnstoff-Na-Salz der Formel

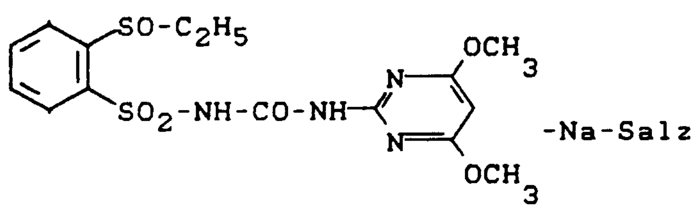

(3a)

gemäß Anspruch 1.

Europäisches Patentamt

# EUROPÄISCHER RECHERCHENBERICHT

Nummer der Anmeldung

EP 92 11 2795

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,X | US-A-4 169 719 (G. LEVITT AT AL.) 2. Oktober 1979 * Tabelle I-A, Verbindung mit R3=-SOCH3; Tabelle I-B, Verbindung mit R3=C2H5SO2- und Verbindung mit R6=-SOC2H5; Tabelle I-C, Verbindung mit R3=-SOC2H5 * | 1-8 | C07D239/47 A01N47/36 C07D239/52 C07D239/56 C07D251/16 C07D251/46 C07D239/42 C07D251/22 |
| D,X | EP-A-0 035 893 (E.I. DU PONT DE NEMOURS AND COMPANY) 16. September 1981 * Seite 30, Zeilen 16-18,22-24; Seite 34, Zeilen 11-13,17-19 * * Ansprüche * | 1-8 | |
| X | US-A-4 655 822 (G. LEVITT ET AL.) 7. April 1987 * Tabellen I-A - I-F, Verbindungen mit R9=Ethyl und n=1 * | 1-8 | |
| D,X | EP-A-0 101 308 (E.I. DU PONT DE NEMOURS AND COMPANY) 22. Februar 1984 * Tabellen I, III-VIII, Verbindungen mit R1=-SOC2H5 | 1-8 | RECHERCHIERTE SACHGEBIETE (Int. Cl.5 ) |
| | | | C07D |
| A | EP-A-0 074 282 (E.I. DU PONT DE NEMOURS AND COMPANY) 16. März 1983 * Tabellen 1,2 * | | |

Der vorliegende Recherchenbericht wurde für alle Patentansprüche erstellt

| Recherchenort | Abschlußdatum der Recherche | Prüfer |
|---|---|---|
| DEN HAAG | 27 NOVEMBER 1992 | DE JONG B.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument